# EUROPÄISCHE PATENTSCHRIFT

(11) **EP 1 711 433 B1**
(45) Veröffentlichungstag und Bekanntmachung des Hinweises auf die Patenterteilung: **04.11.2009**
(21) Anmeldenummer: 05707814.9
(22) Anmeldetag: 17.01.2005
(51) Int. Cl.: C01B 13/18, A61K 6/083, A61K 6/027, A61K 6/04

(54) **DENTALKOMPOSITE AUF DER BASIS VON RÖNTGENOPAKEN SPRÜHFLAMMENSYNTHESE-MISCHOXIDEN**
DENTAL COMPOSITES BASED ON RADIO-OPAQUE SPRAY FLAME SYNTHESIS MIXED OXIDES
COMPOSITE DENTAIRE A BASE D'OXYDES MIXTES, OPAQUES AUX RAYONS X, PRODUITS PAR SYNTHESE PAR PULVERISATION A LA FLAMME

(30) Priorität: 06.02.2004 DE 102004006564; 07.04.2004 DE 102004017125
(43) Veröffentlichungstag der Anmeldung: 18.10.2006
(73) Patentinhaber: Ivoclar Vivadent AG, 9494 Schaan (LI)
(72) Erfinder: BURTSCHER, Peter, A-6830 Rankweil (AT); MÄDLER, Lutz, 28355 Bremen (DE); MOSZNER, Norbert, LI-9495 Triesen (LI); PRATSINIS, Sotriris E., CH-8032 Zürich (CH); RHEINBERGER, Volker, CH-9490 Vaduz (LI)
(74) Vertreter: Fitzner, Uwe
(86) Internationale Anmeldenummer: PCT/EP2005/050252
(87) Internationale Veröffentlichungsnummer: WO 2005/075348

(56) Entgegenhaltungen:
- EP-A- 1 083 146
- EP-A- 1 243 552
- MADLER L ; STARK W J ; PRATSINIS S E: "Simultaneous deposition of Au nanoparticles during flame synthesis of TiO2 and SiO2" JOURNAL OF MATERIALS RESEARCH, Bd. 18, Nr. 1, Januar 2003 (2003-01), Seiten 115-120, XP002330757
- HEIKO SCHULZ, LUTZ MÄDLER, SOTIRIS E. PRATSINIS, PETER BURTSCHER, NORBERT MOSZNER: "Transparent nanocomposites of radiopaque, flame-made Ta2O5/SiO2 particles in an acrylic matrix" ADVANCED FUNCTIONAL MATERIALS, Bd. 15, Nr. 5, 2005, Seiten 830-837, XP002330758 WEINHEIM, GERMANY

## Beschreibung

Die vorliegende Erfindung betrifft die Verwendung mindestens eines durch Sprühflammensynthese hergestellten nanopartikulären Mischoxids (a) von SiO₂ mit röntgenopaken Metalloxiden eines oder mehrerer Elemente ausgewählt aus der Gruppe bestehend aus Y, La, Ta, Ce, Pr, Nd, Sm, Eu, Gd, Tb, Dy, Ho, Er, Tm, Yb, Lu, als röntgenopakem Füllstoff in dentalen Kompositen, die resultierenden Dentalkomposite, deren Herstellung und Verwendung.

### Begriffsdefinitionen

Amorph sind homogene, nichtkristalline Festkörper, bei denen die molekularen Bausteine zwar ähnlich wie in einem Kristall durch eine mehr oder weniger ausgeprägte Nahordnung aneinander gebunden sind, die jedoch die für Kristalle charakteristische Fernordnung, d.h. die regelmäßige Wiederholung eines Basisgitters nicht aufweisen. Entsprechende amorphe Substanzen sind im Gegensatz zu kristallinen Substanzen völlig isotrop. Im Rahmen der vorliegenden Erfindung sind amorph solche Substanzen, die einen Kristallinitätsindex von kleiner 0,1, bevorzugt kleiner 0,05, jeweils bestimmt per Röntgendiffraktometrie, aufweisen (0 = amorph; 1,0 = kristallin). D.h. auf der Grundlage von Röntgen- oder Elektronenbeugung sind solche Substanzen amorph, bei denen im Beugungsspektrum keine auflösbaren Strukturen sichtbar sind. Im übrigen wird auf die einschlägigen Physik- oder Chemie-Lehrbücher verwiesen.

Unter einer homogenen Elementverteilung wird im Rahmen der vorliegenden Erfindung verstanden, daß die Elemente gleichmäßig miteinander vermischt sind, d.h. daß im wesentlichen eine statistische Verteilung der Elemente vorliegt, ohne Bereiche mit Anhäufungen eines einzelnen Elementes. Es liegt demgemäß eine Gleichverteilung der Elemente in den entsprechenden Teilchen vor, die sich in unterschiedlichen Bereichen der Teilchen nicht verändert, also keine Konzentrationsgradienten der Elemente innerhalb der jeweiligen Teilchen vorliegen.

Unter einem sehr geringen organischen Anteil wird im Rahmen der vorliegenden Erfindung verstanden, daß weniger als 0,5 Masse-%, bevorzugt weniger als 0,1 Masse-% oxidierbarerer Kohlenstoff, insbesondere kein organischer Kohlenstoff, d.h. Mengen unterhalb der Nachweisgrenzen, zu finden sind.

Variierbar heißt in Zusammenhang mit der Röntgenopazität der erfindungsgemäßen Substanzen, daß die Röntgenopazität durch die Parameter bei der Herstellung der Mischoxide, d.h. also durch Edukte, Konzentration, Temperatur u.ä., innerhalb gewisser Grenzen durch den Fachmann in geläufiger Weise eingestellt werden kann. Die Röntgenopazität der Komposite auf der Basis der erfindungsgemäßen Mischoxide kann dabei zwischen 50% Al und 800% Al, insbesondere zwischen 100% Al und 400% Al eingestellt werden.

Variierbar heißt in Zusammenhang mit dem Brechungsindex der erfindungsgemäßen Mischoxide, daß der Brechungsindex durch die Parameter bei der Herstellung der Mischoxide, d.h. also durch Edukte, Konzentration, Temperatur u.ä., innerhalb gewisser Grenzen durch den Fachmann in geläufiger Weise eingestellt werden kann. Der Brechungsindex der erfindungsgemäßen Mischoxide kann dabei zwischen 1,46 und 1,70, insbesondere zwischen 1,48 und 1,60 eingestellt werden.

Kugelförmig bedeutet im Rahmen der vorliegenden Erfindung, daß die betreffenden Primärpartikel sphäroid sind und in der Transmissionselektronenmikroskopie (TEM) vergleichbar mir idealen Kugeln keine Vorzugsrichtung oder -kanten zeigen.

Der Ausdruck (Meth)acryl soll im Rahmen der vorliegenden Erfindung sowohl Methacryl als auch Acryl umfassen.

### Stand der Technik

Komposite werden in der Zahnheilkunde vor allem als direktes Füllungsmaterial von Kavitäten, als Befestigungszement oder als Werkstoff für Inlays oder Verblendmaterialien eingesetzt. Sie sind prinzipiell aus einer organischer Monomer- bzw. Polymermatrix und darin eingebetteten Füllstoffen aufgebaut. Die organische Harzmatrix der gegenwärtigen dentalen Füllungskomposite basiert weitgehend auf Dimethacrylaten wie Bis-GMA (ein Additionsprodukt aus Methacrylsäure und Bisphenol-A-diglycidylether), UDMA (ein Additionsprodukt aus 2-Hydroxyethylmethacrylat und 2,2,4-Hexamethylendiisocyanat) oder TEGDMA (Triethylenglycoldimethacrylat). Die Füllstoffe sind meist silikatischer Natur, wobei für einen optimalen Verbund zwischen Harzmatrix und den Füllstoffpartikeln diese mit einem polymerisationsfähigen Silan oberflächenbehandelt sind. Die Füllstoffe gewährleisten vor allem ausreichende mechanischen Eigenschaften, wie hohe Druck-und Biegefestigkeit oder Härte, einen geringen thermischen Ausdehnungskoeffizienten sowie eine Verringerung der Wärmebildung und der Volumenkontraktion bei der Aushärtung, sowie die Einstellung der optischen Eigenschaften und der Röntgenopazität (E. C. Combe, F.J.T. Burke, W.H. Douglas, Dental Biomaterials, Kluwer Academic Publ., Boston 1999, S. 237). Dabei wird die Röntgenopazität vor allem durch den Einbau von Elementen hoher Ordnungszahl (z.B. Ba oder Sr) in den Füllstoffen realisiert. Während bei den Glasfüllstoffen eine Vielzahl von Elementen (Si, Al, B, Ba, Sr oder La) in verschiedenen Anteilen zum Einsatz kommen können und daher der Brechungsindex in einem weiten Bereich (1.46 bis 1.55) variiert werden kann, basieren die bisher verwendeten Nanofüllstoffe im wesentlichen auf Siliziumdioxid (SiO₂), wodurch der Brechungsindex auf einen Bereich von 1.42 bis 1.49 eingeschränkt ist. Eine optimale Übereinstimmung der Brechungsindices von Füllstoff und polymerisierter Matrix sind Voraussetzung für eine hohe Transparenz der Komposite und damit die Grundlage für ästhetische Restaurationen. Mit Monomeren, basierend auf Dimethacrylaten, ist ein breiter Brechungsindexbereich (1.45 bis 1.55) der polymerisierten Matrix einstellbar, die reaktivsten Monomere, wie z.B. Bis-GMA, sind aromatischer Natur mit einem Brechungsindex von ca. 1.55.
Monomere mit einem Brechungsindex von 1.52 bis 1.55 sind erfindungsgemäß bevorzugt.
Die Röntgenopazität von Siliziumoxid ist sehr gering, so daß ein Komposit basierend auf diesem Füllstoff nur eine schwache Röntgenopazität aufweist, was die zahnärztliche Diagnostik erschwert. Röntgenopake Füllstoffe, wie Ytterbiumfluorid oder röntgenopake Gläser haben einen wesentlich höheren Brechungsindex (1.51 bis 1.55) als SiO₂. Die gleichzeitige Verwendung von Füllstoffen mit unterschiedlichem Brechungsindex reduziert jedoch die Transparenz und daher die Ästhetik. Aus diesem Grunde weisen Komposite mit reinen Nanofüllstoffen bei akzeptablen optischen Eigenschaften nur eine geringe Röntgenopazität auf. Röntgenopake, auf Nanofüllstoffen basierende Komposite, sind hingegen zu wenig transparent. Für dentale Komposite geeignete röntgenopake Metalloxid-Füllstoffe sind aus folgendem Stand der Technik bekannt:
Amorphe, kugelförmige anorganische Verbindungen mit einer Teilchengröße von (0,1 bis 1,0 µm) auf der Basis von SiO₂ und mindestens einem Oxid der Elemente der I. bis IV. Gruppe, die über eine naßchemische Synthese hergestellt werden, sind in der DE 32 47 800 beschrieben. Darauf basierende dentale Komposite beinhaltet die DE 40 29 230.
In der DE 195 08 586 werden Füllstoffe beschrieben, die ausgehend von einem SiO₂-Kern durch Beschichtung mit einem Oxid eines Elementes der I. bis IV. Gruppe Sol-Gel-chemisch erhalten werden. Solche Füllstoffe sind auch in der DE 197 41 286 erwähnt.
Polymerisierbare Metalloxidpartikel, die einen Kern-Schale-Aufbau zeigen, sind in der DE 198 46 660 offenbart. Solche Füllstoffe sind durch Oberflächenmodifizierung von z.B. käuflichen SiO₂-Partikeln mit Metallalkoxiden zugänglich.
Oxidpartikel, die sich als Füllstoff für Dentalmaterialien eignen, mit einem Kern aus einem beliebigen Metall- oder Metalloidoxid des Periodensystems, einer im Kern verteilten beliebigen Dotierkomponente und einer den Kern umgebenden Hülle sind in der EP 1 243 552 beschrieben. Diese Partikel werden so hergestellt, daß zunächst die Dotierung in einem pyrogenen Prozeß über ein Aerosol in der Kern eingebracht wird, der dann nachfolgend umhüllt wird.
In der EP 1 236 459 werden lichthärtende Dentalkomposite mit ausgezeichneten Handlingeigenschaften und Bruchzähigkeit beschrieben, die einen Füllstoff enthalten, der eine Mischung von größenangepaßten Partikeln von irregulärer Form (0,1 bis 1,0 µm), kugelförmigen Partikeln (0,1 bis 5,0 µm) und sehr kleinen Partikeln (kleiner 0,1 µm) darstellt. Als Stoffe für die Füllstoffpartikel werden z.B. SiO₂ZrO₂ oder SiO₂-TiO₂ erwähnt.

Nanopartikuläre Metalloxid- bzw. Mischoxid-Füllstoffe sind für die Anwendung in Dentalmaterialien, z.B. als Füllungskomposite, von besonderem Interesse, da sie einerseits die Kombination verschiedener Eigenschaften ermöglichen, z.B. hohe Biegefestigkeit, geringe Abrasivität und optimaler Röntgenopazität, andererseits aufgrund ihrer geringen Partikelgröße (kleiner 100 nm) die Herstellung von transparenten bzw. transluzenten Werkstoffen, d.h. von Materialien mit zahnähnlichen ästhetischen Eigenschaften, ermöglichen (vgl. "Nanotechnology for Dental Composites" N. Moszner, S. Klapdohr, Intern. J. Nanotechn., 1 (2004) 130-156). Solche nanopartikulären Metalloxid-Füllstoffe lassen sich beispielsweise auf naßchemischen Wege durch hydrolytische Kondensation (Sol-Gel-Prozeß) von einzelnen Metallalkoxiden bzw. deren Mischungen oder durch Flammenpyrolyse von geeigneten Precursor-Verbindungen wie Metallalkoxiden, -salzen oder -halogeniden herstellen. Dabei sind die physikalischen und chemischen Eigenschaften der Nanopartikel unter anderem von deren chemischer Zusammensetzung und Morphologie, ihrer Partikelgröße bzw. -größenverteilung und der Oberflächenmodifizierung abhängig.
Die Verwendung von Kombinationen von Nanopartikeloxiden, bei der mindestens ein Oxid eine nanopartikuläre und röntgenopake Metalloxid-Komponente darstellt, als dentalem Füllstoff ist aus dem folgenden Stand der Technik bekannt:
Nanoskaliges, pyrogen hergestelltes Yttrium-Zirkon-Mischoxid mit einer spezifischen Oberfläche von 1 bis 800 m²/g ist in der DE 101 38 573 als keramischer Grundstoff für Dentalwerkstoffe beschrieben.
In der DE 100 18 405 werden sphärische oxidische Partikel mit einer Partikelgröße von 5 bis 10000 nm beschrieben, die 0,1 bis 99,9 Gew.-% eines Oxids der Elemente Titan, Aluminium, Zirkonium, Yttrium oder Silicium und mindestens ein weiteres Oxid der Lanthanoide enthalten, wobei die Partikel einen Kern-Schale-Aufbau oder eine homogene Verteilung der Metalloxide zeigen können.
Dentalmaterialien auf Basis von Nanopartikel-Füllstoffen beschreiben die WO 01/30304, WO 01/30305, WO 01/30306, WO 01/30307 in den neben SiO₂-Partikeln zusätzlich als röntgenopaker Füllstoff nanopartikuläre Schwermetalloxide von Metallen mit Ordnungszahl größer 28 enthalten sind. Als besonders bevorzugte Oxide werden z.B. La-, Zn-, Sn-, Y-, Yb-, Ba- und Sr-Oxid oder deren Kombinationen angegeben, wobei die bevorzugte Partikelgröße kleiner 60 nm liegt. Mischoxide aus SiO₂ und Yb₂O₃ we rden nicht erwähnt. Zusätzlich ist angegeben, daß die beschriebenen Schwermetalloxid-Komponenten ein Teil der Beschichtung der SiO₂-Partikel darstellen kann. Weiterhin werden darüber hinaus amorphe, nanopartikuläre Cluster beansprucht, die bevorzugt aus den Nichtschwermetaloxiden z.B. SiO₂ oder As₂O₃ und den Oxiden der Schwermetalle z.B. La, Zn, Sn, Y, Yb, Ba oder Sr zugänglich sind. Dabei beschreibt der Begriff Cluster die Art des Zusammenschlusses der Partikel, wobei die Schwermetalloxide in den Clustern als individuelle Partikel, als Beschichtung der Nichtschwermetalloxid-Partikel oder als Region in den Nichtschwermetalloxid-Partikeln vorliegen. Außerdem kann das Schwermetalloxid in den Nichtschwermetalloxid-Partikeln als feste Lösung (z.B. als kontinuierliches Glas) sowie als Präzipitat in einer zweiten Phase vorliegen. Weiterhin wird erwähnt, daß die Cluster im wesentlichen keine Kristallinität zeigen, d.h. die beanspruchten Füllstoffe haben bevorzugt einen Kristallinitätsindex kleiner 0,1 (0 = amorph; 1,0 = kristallin). In den Beispielen sind als Metalloxide SiO₂ und ZrO₂ aufgeführt, wobei die Cluster so hergestellt werden, daß z.B. käufliche SiO₂-Partikel Sol-Gel-chemisch mit Zirkonylacetat modifiziert wurden.
Ta₂O₅-SiO₂-Partikel mit einem Durchmesser zwischen 50 bis 100 nm werden in der US 6,417,244 B1 beansprucht. Dabei erfolgt die Partikelsynthese ausgehend von Dispersionen von monodispersen SiO₂-Partikeln (10 bis 20 nm) und Ta₂O₅-Partikeln (1 bis 2 nm).
In der WO 99/17716 sind niedrigviskose Dentalmaterialien beschrieben, die nichtagglomerierte Nanopartikel von 1 bis 100 nm enthalten, wobei als Füllstoff u.a. pyrogene Kieselsäure, Tantal- und Nioboxid und deren Mischungen angegeben werden.

In der US 2002/0002214 A1 werden kationisch polymerisierbare Zusammensetzungen beschrieben, bei denen als röntgenopake Füllstoffe Oxide, deren Mischungen bzw. Mischoxide der Elemente La, Zn, Ta, Sn, Zr, Y, Yb, Ba, Sr mit Oxiden der Elemente Al, B oder Si, die über den Sol-Gel-Prozeß oder über eine Schmelze zugänglich sind. Eine Partikelgröße ist für die Füllstoffe nicht angegeben.
Füllstoffe auf der Basis von Ormocermischoxidpartikeln der Elemente Ti, Zr, Y, La, Ta und Al mit Si- oder Ti-organischen Komponenten, die organische Gruppen enthalten und sichtbares Licht im Bereich von 360 bis 830 nm nicht streuen, werden in der GB 2.304.720 beschrieben.

Die in dem voranstehend zitierten Stand der Technik beschriebenen nanopartikulären Füllstoffe, in denen SiO₂ mit einem röntgenopaken Metalloxid kombiniert wurde, tragen aufgrund ihrer 2-Phasen-Morphologie (z.B. Kern-Schale, Dotierung, Mischung der Oxide) und der damit verbundenen nicht homogenen Elementverteilung im Füllstoff sowie der teilweise kristallinen Struktur nur unzureichend zu transparenten Eigenschaften im Komposit bei. Zudem ist eine Anpassung des Brechungsindex der Füller an den der Matrix nur eingeschränkt möglich. Darüber hinaus zeigen die nanopartikulären, nichtkugelförmigen Füllstoffe meist eine extreme, jedoch kaum beeinflußbare Verdickungswirkung.

Weiterer Nachteil der naßchemischen Herstellungsverfahren von Mischoxiden ist es, daß bei nicht vollständiger Trocknung - welche äußerst aufwendig ist - immer kleine Reste der Lösungsmittel verbleiben, die dann beim nachfolgenden Calcinieren zu einer Verfärbung der Partikel und damit auch des Dentalkomposites führen können. Solcherart verfärbte Produkte können aber aus ästhetischen Gründen in aller Regel nicht verwendet werden und stellen somit Ausschuß dar.

Aus der EP-A-1 243 552 A1 ist der Einsatz für den jeweiligen Polierzweck maßgeschneiderter Mischoxidpartikel für das chemisch-mechanische Polieren von Halbleitersubstraten und von auf ihnen aufgebrachten Schichten bekannt. Die dargestellten Oxidpartikel finden demgemäß Verwendung bei der Behandlung von Halbleitersubstraten.

### Beschreibung der Erfindung

Der Erfindung liegt die Aufgabe zugrunde, Dentalkomposite bereitzustellen, die sich im Vergleich zum Stand der Technik durch gute Röntgenopazität, bei hoher Transparenz und geringer Eigenfärbung durch den Füllstoff auszeichnen, eine Variation der Röntgenopazität, des Brechungsindex und der Verdickungswirkung des Füllstoffes gestatten und zur Herstellung von Zementen, Verblendmaterialien und vor allem von Füllungskompositen für dentale Zwecke geeignet sind.
Die Aufgabe wird erfindungsgemäß durch Dentalkomposite enthaltend mindestens ein nanopartikuläres Mischoxid erhältlich durch Sprühflammensynthese (a) von SiO₂ mit röntgenopaken Metalloxiden eines oder mehrerer Elemente ausgewählt aus der Gruppe bestehend aus Y, La, Ta, Ce, Pr, Nd, Sm, Eu, Gd, Tb, Dy, Ho, Er, Tm, Yb, Lu, wobei die Mischoxide durch Sprühflammensynthese hergestellt werden, und die eine amorphe Struktur, eine homogene Elementverteilung, einen sehr geringen organischen Anteil, eine variierbare Röntgenopazität bzw. Brechungsindex sowie eine kugelförmige Partikelgestalt und durch Agglomerierung eine verringerte Verdickungswirkung zeigen.

Die erfindungsgemäßen Mischoxide weisen eine sehr homogene Elementverteilung auf, welche mit bisherigen naßchemischen Verfahren nicht erreichbar war.
Durch den Wegfall eines Lösungsmittels, wie sie in naßchemischen Verfahren notendig sind, entfällt, neben den oben angeführten Nachteilen ferner dessen nachträgliche Entfernung, und eine Agglomeratbildung, die zu größeren Sekundärteilchen mit breiterer Korngrößenverteilung und folglich geringerer Transparenz führt, wird ausgeschlossen.
Die Herstellung der erfindungsgemäßen Mischoxide ist darüber hinaus aufgrund dessen, daß es ein kontinuierlicher Prozeß ist, wirtschaftlich günstiger und einfacher durchzuführen.

Vorteilhaft ist auch, daß durch die Wahl der Komponenten bei der Sprüflammensynthese der Brechungsindex der Primärpartikel bei extrem kleiner Korngröße (kleiner als 50 nm) und enger Korngrößenverteilung einstellbar ist.
Ebenso sind durch das Herstellungsverfahren der erfindungsgemäßen Mischoxide deren Korngrößen gezielt einstellbar.

Bei der Sprühflammenpyrolyse besteht der Reaktor aus einer Mehrstoffdüse, welche konzentrisch von einer Hilfsflamme umgeben ist (L. Mädler, H. K. Kammler, R. Mueller, und S. E. Pratsinis, "Controlled synthesis of nanostructured particles by flame spray pyrolysis," Journal of Aerosol Science, vol. 33, pp. 369-389, 2002; L. Mädler, W. J. Stark, und S. E. Pratsinis, "Flame-made ceria nanoparticles," Journal of Materials Research, vol. 17, pp. 1356-1362, 2002; R. Mueller, L. Mädler, und S. E. Pratsinis, "Nanoparticle synthesis at high production rates by flame spray pyrolysis," Chemical Engineering Science, vol. 58. pp. 1969-1976, 2003). Die Hilfsflamme dient zum Zünden des Sprays und wird mit einem brennbaren Gasgemisch (z.B. CH₄/O₂, H₂/O₂) gespeist. Die Mehrstoffdüse dispergiert mindestens eine brennbare Flüssigkeit in fein verteilte Tröpfchen, die im Idealfall zwischen 1 µm und 100 µm groß sind. Zusätzlich kann ein Hülluftstrom außerhalb der Schutzflamme zugeschaltet werden. Die in der Sprühflamme gebildeten Partikel werden durch Vermischen mit Umgebungsluft abgekühlt. Andere Abkühlungsmethoden sind jedoch ebenfalls möglich, z.B. Einspritzen von Flüssigkeiten hoher Verdampfungsenthalpie oder Nozzlequenching (K. Wegner and S. E. Pratsinis, "Nozzle-quenching process for controlled flame synthesis of titania nanoparticles," AIChE Journal, vol. 49, pp. 1667-1675, 2003). Danach werden die gasgetragenen Partikel auf einen geeigneten Filter abgeschieden und von diesem abgereinigt Die dispergierte Flüssigkeit enthält sowohl den Brennstoff als auch die Metalloxid-Vorläufersubstanzen (Precursor). Als Precursor für SiO₂ sind vor allem Tetraalkoxysilane, wie z.B. Trimethyl- oder Tetraethylsilan bevorzugt. Im Falle der röntgenopake Metalloxide der Elemente ausgewählt aus der Gruppe Y, La, Ta, Ce, Pr, Nd, Sm, Eu, Gd, Tb, Dy, Ho, Er, Tm, Yb und Lu eignen sich entsprechende Metallsalze z.B. Nitrate, Halogenide oder Carboxylate, wie z.B. Formiate, Acetate, Oxalate, Triflate, 2-Ethylhexanoate sowie Naphthenate, Metallalkoxide und Metallchelate, wie z.B. Chelate von Acetylaceton, Acetessigester, Dimethylglyoxim, Salicylaldehyd, 8-Hydroxychinolin oder o-Phenanthrolin, die in einem geeigneten Lösungsmittel gelöst werden oder durch entsprechende Umsetzungen in eine gut homogen lösliche Metallverbindung überführt werden. Die Precursorflüssigkeit sollte bevorzugt eine homogene Lösung sein, wobei prinzipiell jedoch auch Emulsionen möglich sind. Wird eine Mehrstoffdüse mit mehreren Flüssigkeitszuführungen verwendet, können der Brennstoff und die jeweiligen Metalloxidprecursor auch getrennt zerstäubt werden. Im Idealfall besteht der Brennstoff aus einem organischen Lösungsmittel in dem der (oder die) Metalloxidprecursor gelöst vorliegen. Als Lösungsmittel/Brennstoff sind vor allem bevorzugt Alkohole, organische Säuren sowie aromatische und/oder aliphatische Kohlenwasserstoffe.
Die Partikelbildung läßt sich so vorstellen, daß die Mehrstoffdüse die Precursor-Lösungsmittel/Brennstoff-Flüssigkeitsmischung in feine Tröpfchen zerstäubt. In der Flamme werden diese Tröpfchen sehr hohen Temperaturen (1500-2500 K) ausgesetzt. Dies führt zur Verdampfung und anschließender Reaktion der/des Precursor(s) und des Lösungsmittels/Brennstoffs (Verbrennung). Die Verdampfung und Freisetzung kann durch Tropfenexplosionen aufgrund von Überhitzung der Tropfen beeinflußt werden. Ebenfalls ist ein Zerfall der/des Precursor(s) (Gas- oder Flüssigphase) oder Sublimierung vor der Reaktion möglich. Anschließend bilden sich aufgrund der Reaktion erste Moleküle und Molekülcluster der Metalloxide. Durch Koagulation dieser ersten sub-nanometer Partikel entstehen größere Partikel, die in diesem Hochtemperaturbereich der Flamme noch flüssig sind und daher homogen zusammenschmelzen. Die Partikel erfahren dadurch einen Wachstumsprozeß. Nachdem alle exothermen Reaktionen abgelaufen sind, kühlt sich die Prozeßumgebung ab, und ein Zusammenschmelzen der Partikel wird unterdrückt Dies fuhrt zum "Einfrieren" der Partikel, die damit ihre Zusammensetzung und Form nicht mehr ändern.
In Abhängigkeit von der Art und Menge der/des verwendeten Metalloxidprecursor(s), des Brennstoff/Lösungsmittels, der Menge und Art des Zerstäubungsgases und Menge der Flüssigkeitszufuhr können chemische Zusammensetzung, die Morphologie, die Partikelgröße bzw. -größenverteilung und Produkteigenschaften der gebildeten SiO₂-Mischoxidpartikel gezielt kontrolliert werden. Die Partikelgröße und damit deren spezifische Oberfläche läßt sich durch die Menge und den Energieinhalt der Precursor-Lösungsmittel/Brennstoff-Flüssigkeitsmischung sowie die Art und Menge an Dispersionsgas variieren (vgl. L. Mädler, W. J. Stark, und S. E. Pratsinis, "Flame-made ceria nanoparticles," Journal of Materials Research, vol. 17, pp. 1356-1362, 2002; L Mädler, H. K. Kammler, R. Mueller, und S. E. Pratsinis, "Controlled synthesis of nanostructured particles by flame spray pyrolysis," Journal of Aerosol Science, vol. 33, pp. 369-389, 2002). Die Phasenstruktur (W. J. Stark, L. Mädler, M. Maciejewski, S. E. Pratsinis, und A. Baiker, "Flame synthesis of nanocrystalline ceria-zirconia: effect of carrier liquid," Chemical Communications, pp. 588-589, 2003) bzw. die Kontrolle über die Morphologie, d.h. die Bildung von hohlen und/oder kompakten Teilchen (L. Mädler and S. E. Pratsinis, "Bismuth oxide nanoparticles by flame spray pyrolysis," Journal of the American Ceramic Society, vol. 85, pp. 1713-1718, 2002), läßt sich durch das Lösungsmittel beeinflussen bzw. realisieren.
Die Mischoxide (a) weisen erfindungsgemäße bevorzugt eine mittlere Primärpartikelgröße von 3 bis 100nm, insbesondere 5 bis 40nm, bestimmt durch Messung der BET-Oberfläche auf.

Die durch Sprühflammensynthese hergestellten nanopartikulären Mischoxide von SiO₂ mit röntgenopaken Metalloxiden eines oder mehrerer Elemente ausgewählt aus der Gruppe bestehend aus Y, La, Ta, Ce, Pr, Nd, Sm, Eu, Gd, Tb, Dy, Ho, Er, Tm, Yb, Lu können als röntgenopake Füllstoffe verwendet werden. Bevorzugt sind Mischoxide von SiO₂ mit einem röntgenopaken Metalloxid von Yb, insbesondere SiO₂/Yb₂O₃.
Die Mischoxide können als röntgenopake Füllstoffe in quasi beliebigen Werkstücken, bei denen die Gegenwart eines röntgenopaken Füllstoffs, z.B. zur Analytik, vorteilhaft ist, eingesetzt werden. Bevorzugt werden sie in Dentalkompositen verwendet.

Für die Herstellung der erfindungsgemäßen Dentalkomposite werden die durch Sprühflammensynthese hergestellten röntgenopaken Mischoxidnanofüllstoffe in geeigneten polymerisierbaren Matrixharzen dispergiert, anschließend mit dem Photoinitiatorsystem und gegebenenfalls weiteren Additiven versetzt und durch thermische oder lichtinduzierte Polymerisation ausgehärtet. Darüber hinaus kann der Füllgrad eines nanogefüllten Komposits noch weiter erhöht werden, indem die Nanofüller in einen vorpolymerisierten Füller eingearbeitet werden.
Als radikalisch polymerisierbare Matrixmonomere können kommerziell verfügbare Verdünnermonomere wie Mono(meth)acrylate, z.B. Methyl-, Ethyl-, Butyl-, Benzyl-, Furfuryl- oder Phenyl(meth)acrylat sowie die als Vernetzermonomere bekannten mehrfunktionellen Acrylate bzw. Methacrylate wie z.B. Bisphenol-A-di(meth)acrylat, Bis-GMA, UDMA, Di-, Tri- oder Tetraethylenglycoldi(meth)acrylat, 1,10-Decandioldi(meth)acrylat, Trimethylolpropantri(meth)acrylat, Pentaerythrittetra(meth)acrylat, sowie Butandioldi(meth)acrylat oder 1,12-Dodecandioldi(meth)acrylat eingesetzt werden. Als radikalisch polymerisierbare Oligomere oder Polymere, die endständige und/oder seitenständige radikalisch polymerisierbare Gruppen tragen, lassen sich beispielsweise radikalisch polymerisierbare α,ω-(meth)acryloyl-terminierte Polyester-, Polyether-, Polyepoxid-Amin- oder Polyurethan-Telechele oder Kieselsäurepolykondensate, die beispielsweise durch hydrolytische Kondensation von Silanen, die radikalisch polymerisierbare Gruppen, bevorzugt z.B. Methacryl- oder Acrylgruppen tragen, verwenden. Solche Kieselsäurepolykondensate sind auch in der DE 44 16 857 C1 oder der DE 41 33 494 C2 beschrieben.
Als Matrixmonomere für kationische Photopolymerisate kommen vor allem in Frage kationisch polymerisierbare Verdünner- oder Vernetzermonomere wie z.B. Glycidylether oder cycloaliphatische Epoxide, cyclische Ketenacetale, Vinylether, Spiro-orthocarbonate, Oxetane oder bicyclische Orthoester. Beispiele hierfür sind: Triethylenglykoldivinylether, Cyclohexandimethanoldivinylether, 2-Methylen-1,4,6-trioxaspiro[2.2]nonan, 3,9-Dimethylen-1,5,7,11-tetraoxaspiro[5.5]undecan, 2-Methylen-1,3-dioxepan, 2-Phenyl-4-methylen-1,3-dioxolan, Bisphenol-A-diglycidylether, 3,4-Epoxycyclohexylmethyl-3,4-epoxycyclohexancarboxylat, Bis(-(3,4-epoxycyclohexylmethyl)adipat, Vinylcyclohexendioxid, 3-Ethyl(3-hydroxymethyloxetan, 1,10,-Decandiylbis(oxymethylen)bis(3-ethyloxetan) oder 3,3-(4-Xylylendioxy)-bis-(methyl-3-ethyloxetan). Als kationisch polymerisierbare Matrixsysteme eignen sich auch Kieselsäurepolykondensate, die beispielsweise durch hydrolytische Kondensation von Silanen, die kationisch polymerisierbare Gruppen, bevorzugt z.B. Epoxid-, Oxetan-, Spiroorthoester oder Vinylethergruppen tragen. Solche Kieselsäurepolykondensate sind beispielsweise in der DE 41 33 494 C2 oder US 6,096,903 beschrieben.
Zur Initiierung der radikalischen Polymerisation werden Polymerisationsinitiatoren, vorzugsweise thermische und/oder Photoinitiatoren den erfindungsgemäß eingesetzten Zusammensetzungen zugegeben. Bevorzugte Beispiele für thermische Initiatoren sind die bekannten Peroxide, wie z.B. Dibenzoylperoxid, Dilaurylperoxid, tert.-Butylperoctoat oder tert.-Butylperbenzoat sowie Azobisisobutyroethylester oder Azoisobutyronitril (AIBN), Benzpinakol oder 2,2-Dimethylbenzpinakol. Beispiele für geeignete Photoinitiatoren sind Benzophenon, Benzoin sowie deren Derivate oder α-Diketone oder deren Derivate wie 9,10-Phenanthrenchinon, 1-Phenyl-1,2-propandion, Diacetyl- oder 4,4-Dichlorbenzil. Besonders bevorzugt werden Campherchinon und 2,2-Methoxy-2-phenyl-acetophenon und besonders bevorzugt α-Diketone in Kombination mit Aminen als Reduktionsmittel, wie z.B. N-Cyanoethyl-N-methylanilin, 4-(N,N-Dimethylamino)-benzoesäureester, N,N-Dimethylaminoethylmethacrylat, N,N-Dimethyl-sym.-xylidin oder Triethanolamin, eingesetzt. Darüber hinaus sind auch Acylphosphine, wie z.B. 2,4,6-Trimethylbenzoyldiphenyl- oder Bis(2,6-dichlorbenzoyl)-4-N-propylphenylphosphinoxid besonders geeignet. Für die duale Aushärtung von radikalisch und kationisch polymerisierbaren Systemen eignen sich besonders Diaryliodonium- oder Triarylsulfoniumsalze, wie z.B. Triphenylsulfoniumhexafluorophosphat oder -hexafluoroantimonat.
Als Initiatoren für eine bei Raumtemperatur durchgeführte Polymerisation werden Redox-Initiatorkombinationen, wie z.B. Kombinationen von Benzoyl- oder Laurylperoxid mit N,N-Dimethyl-sym.-xylidin oder N,N-Dimethyl-p-toluidin, verwendet.
Für einen optimalen Verbund zwischen Matrix und Füllerpartikeln, werden diese üblicherweise mit Silanen oberflächenbehandelt, wobei die Silane geeignete polymerisationsfähige Gruppen, wie (Meth)acryl-, Vinyl-, Oxetan- oder Epoxidgruppen enthalten.
Weiterhin können die erfindungsgemäß eingesetzten Zusammensetzungen zur Verbesserung der mechanischen Eigenschaften mit weiteren organischen oder anorganischen Partikeln oder Fasern gefüllt werden. Bevorzugte anorganische partikuläre Füllstoffe sind nanopartikuläre oder mikrofeine Füllstoffe, wie pyrogene Kieselsäure oder Fällungskieselsäure sowie Makro- oder Minifüllstoffe, wie Quarz-, Glaskeramik- oder Glaspulver mit einer durchschnittlichen Teilchengröße von 0,01 bis 5 µm sowie röntgenopake Füllstoffe, wie z.B. Ytterbiumtrifluorid. Darüber hinaus können auch Titan, Glasfasern, Polyamid- oder Kohlenstoff-Fasern eingesetzt werden. Schließlich können die erfindungsgemäß eingesetzten Zusammensetzungen weitere Additive enthalten, wie z.B. Stabilisatoren, Aromastoffe, mikrobiozide Wirkstoffe, optische Aufheller, Weichmacher oder UV-Absorber.

Eine bevorzugte erfindungsgemäß eingesetzte Zusammensetzung enthält:
(a) 5 bis 90, insbesondere 10 bis 70 Gew.-% mindestens eines nanopartikulären Mischoxids (a) von SiO₂ mit röntgenopaken Metalloxiden eines oder mehrerer Elemente ausgewählt aus der Gruppe bestehend aus Y, La, Ta, Ce, Pr, Nd, Sm, Eu, Gd, Tb, Dy, Ho, Er, Tm, Yb, Lu, wobei die Mischoxide durch Sprühflammensynthese hergestellt wurden, sowie
(b) 0 bis 80, insbesondere 0 bis 50 Gew.-%, bezogen auf die Zusammensetzung, mindestens ein Matrixmonomer,
(c) 0,1 bis 5, insbesondere 0,2 bis 2,0 Gew.-%, bezogen auf die Zusammensetzung, Polymerisationsinitiator, und/oder
(d) 0 bis 90, insbesondere 0 bis 80 Gew.-%, bezogen auf die Zusammensetzung an weiteren Füllstoffen,
(e) 0,0001 bis 0,5, insbesondere 0,001 bis 0,3 Gew.-%, bezogen auf die Zusammensetzung, Farbmittel,
(f) 0,001 bis 2,0, insbesondere 0,1 bis 1,0 Gew.-%, bezogen auf die Zusammensetzung, weitere Additive,
wobei sich die Anteile der Komponenten (a) bis (f) zu 100 Gew.-% addieren.

Eine besonders bevorzugte erfindungsgemäß eingesetzte Zusammensetzung enthält:
(a) 5 bis 90, insbesondere 10 bis 70 Gew.-% mindestens eines nanopartikulären Mischoxids (a) von SiO₂ mit röntgenopaken Metalloxiden eines oder mehrerer Elemente ausgewählt aus der Gruppe bestehend aus Y, La, Ta, Ce, Pr, Nd, Sm, Eu, Gd, Tb, Dy, Ho, Er, Tm, Yb, Lu, wobei die Mischoxide durch Sprühflammensynthese hergestellt wurden, sowie
(b) 0 bis 80, insbesondere 0 bis 50 Gew.-%, bezogen auf die Zusammensetzung, mindestens ein Matrixmonomer,
(c) 0,1 bis 5, insbesondere 0,2 bis 2,0 Gew.-%, bezogen auf die Zusammensetzung, Polymerisationsinitiator, und/oder
(d) 0 bis 90, insbesondere 0 bis 80 Gew.-%, bezogen auf die Zusammensetzung an weiteren Füllstoffen,
wobei sich die Anteile der Komponenten (a) bis (d) zu 100 Gew.-% addieren.

In einer weiteren Ausführungsform sind die Dentalkomposite der vorliegenden Erfindung frei von Apatiten.

Weiterhin bevorzugt sind Dentalkomposite, die eine Röntgenopazität von 50 bis 800 % Al, insbesondere 100 bis 400 % Al, Mischoxide (a) mit einer mittleren Primärpartikelgröße von 3 bis 100 nm, bestimmt durch Messung der BET-Oberfläche, einen Brechungsindex von 1,46 bis 1,70, insbesondere 1,48 bis 1,60 und/oder einen organischen Anteil von weniger als 0,5 Masse-%, bevorzugt weniger als 0,1 Masse-% oxidierbaren Kohlenstoff, insbesondere keinen organischen Kohlenstoff aufweisen.

Die erfindungsgemäßen Dentalkomposite können z.B. verwendet werden, indem sie in Dentalkavitäten direkt eingefüllt werden oder auf die Oberflächen von Zähnen aufgetragen werden. Die Dentalkomposite können in eine Form, insbesondere eine Dentalkavität oder Prothesenrohform, eingebracht und gehärtet werden.

Weiterhin eignen sich die Dentalkomposite für Dentalfüllungen, zur Oberflächenversiegelung von Zähnen sowie zur Restauration von Zähnen, zur Herstellung von Zahnprothesen, Dentalbrücken und/oder ähnlichen Zahnersatzstücken sowie zur Füllung oder Beschichtung von Substraten und als Adhäsiv, Zement- und/oder Verblendungmaterialien Zement für Dentalanwendungen.

In einer weiteren Verwendungsmöglichkeit kann zur Herstellung von Inlays oder Onlays ein Abdruck einer Zahnkävität angefertigt werden, auf deren Basis dann ein passendes Inlays oder Onlays angefertigt wird.
Die erfindungsgemäßen Komposite können darüber hinaus zur Füllung oder Beschichtung von beliebigen Substraten, insbesondere jedoch von Zähnen verwendet werden.
Die Komposite werden normalerweise derart verwendet, daß sie nacheinander
(I) aufgetragen oder in eine Form gefüllt werden,
(II) gegebenenfalls ge- bzw. verformt werden,
(III) gegebenenfalls teilgehärtet werden,
(IV) gegebenenfalls nachbearbeitet werden,
(V) ausgehärtet werden und
(VI) gegebenenfalls abschließend nachbearbeitet werden.

Die Erfindung wird im folgenden anhand der Figuren und anhand von Beispielen näher erläutert.

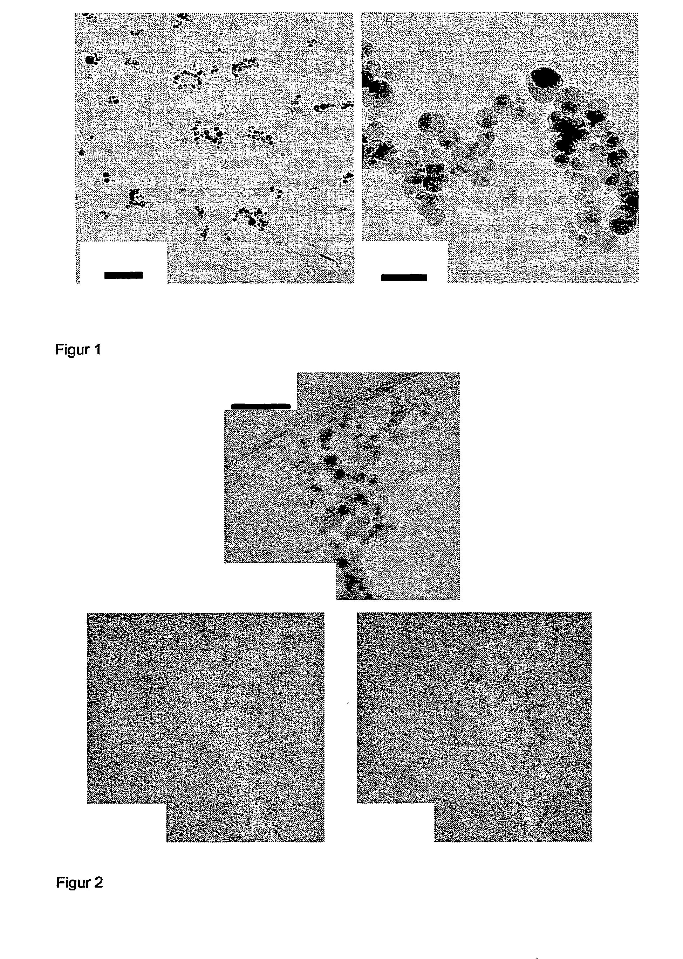

Figur 1 zeigt (Maßstab in nm) Aufnahmen unterschiedlicher Vergrößerung (links 10-tausendfach, rechts 50-tausendfach) mittels Transmissions-Elektronen-Mikroskopie (TEM) eines Yb₂O₃/SiO₂-Mischoxidpulvers (30 Masse-% Yb₂O₃) hergestellt durch Sprühflammensynthese. Die Übersichtsaufnahmen zeigen die morphologische Homogenität des Produktes, das aus teilweise aggregierten Primärpartikeln besteht. Die Primärpartikel des Pulvers zeichnen sich durch ihre kugelförmige Gestalt aus.

Figur 2 zeigt eine Elektronenmikroskopaufnahme eines Yb/Si-Mischoxides (50 Masse-% Yb₂O₃) aus der Sprühflammensynthese (oberstes Bild). Auch bei diesem Yb₂O₃-Masseanteil sind die Primärpartikel kugelförmig. Die unteren Bilder zeigen das Elektronenspektroskopiebild (ESI) für die Energieabsorptionskanten von Yb-M (1,53 und 1,58 keV) (Mitte) und Si-K (1,84 keV) (unterstes Bild). Im Vergleich der beiden unteren Bilder wird deutlich, daß die Yb- und Si-Atome sehr homogen verteilt sind. Diese homogene Verteilung besteht auch innerhalb der einzelnen Primärpartikel auf sehr kurzen Längenskalen.

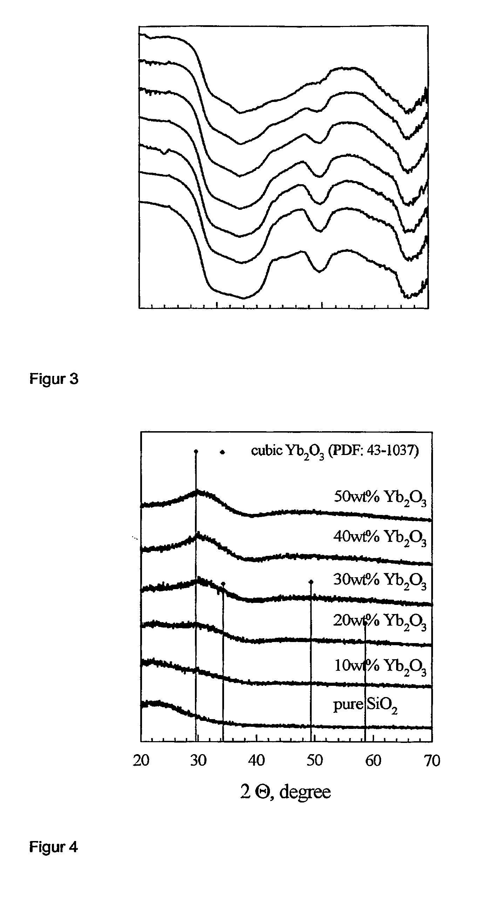

Figur 3 zeigt DRIFTS-Spektren (Diffuse Reflektionsinfrarotspektroskopie) von Yb/Si-Mischoxiden mit unterschiedlichem Yb₂O₃ Gehalt (0 bis 50 Masse-%) hergestellt mittels der Sprühflammensynthese. Beim Zusatz von Ytterbium zeigt sich ein breites Absorptionssignal zwischen 1000 und 900 cm⁻¹, dessen Intensität sich mit zunehmenden Ytterbiumgehalt erhöht. Diese breite Absorptionsbande entspricht degenerierten Vibrationsmoden, die durch die Präsenz von Ytterbium hervorgerufen wird. Die erhöhte Intensität der Absorption mit steigendem Ytterbiumgehalt belegt die atomare Verteilung des Ytterbiums im Pulver.

Figur 4 zeigt XRD (Röntgendiffraktogramme) von Yb/Si Mischoxiden mit unterschiedlichem Yb₂O₃-Gehalt (0 bis 50 Masse-%) hergestellt mittels Sprühflammensynthese. Das Beugungsmuster von reinem kubischem Yb₂O₃ ist durch vertikale Linien dargestellt. Keines der Pulver zeigt ausgeprägte Röntgenbeugungsmuster; demgemäß sind alle Proben röntgenamorph. Es konnten weder Yb₂O₃-Kristalle noch Ytterbiumsilikate nachgewiesen werden.

### Beispiele

Die in den Beispielen angeführten Meßdaten erfolgten entsprechend der EN ISO 4049 (2000) "Zahnheilkunde - Füllungs-, restaurative und Befestigungskunststoffe":

### Biegefestigkeit und Biegemodul:

In entsprechenden Stahlformen werden mit der Kompositpaste Prüfkörper mit einer Abmessung 25mm*2mm*2mm hergestellt und mit einer dentalen Strahlungsquelle Spectramat (Ivoclar Vivadent) jeweils pro Seite 3 Minuten, d.h. 2*3 Minuten ausgehärtet. Nach 24 Stunden Wasserlagerung der Prüfkörper bei 37°C wurden die mechanischen Eigenschaften mit einer Universal-Prüfmaschine Z010 der Firma Zwick bestimmt.

### Transparenz:

In einem ChromaMeter CT-310 (Minolta) wird ein Weißlichtstrahl zur Kalibrierung durch eine mit Wasser gefüllte Küvette (Schichtdicke: 2mm) geschickt, was einer Transparenz von 100% entspricht. Dann wird die Küvette durch einen Kompositprüfkörper (Schichtdicke: 1mm) ausgetauscht und das durchgelassene Licht im Vergleich zur Wasserküvette gemessen, was dann der Transparenz des Komposites entspricht.

### Röntgenopazität:

Von einem Kompositprüfkörper mit einer Schichtdicke von 2mm und einer standardisierten Al-Treppe mit einer Stufenhöhe von jeweils 0,5mm wird zusammen mittels einer dentalen Röntgenkamera eine Röntgenaufnahme angefertigt und die Schwärzung der Kompositprobe und Al-Treppe verglichen. Die Schwärzung von 2mm Al entsprechen einer Röntgenopazität von 100% Al.

### Beispiel 1: Synthese der Mischoxide der Elemente Si und Yb

Es wurde eine Zweistoffdüse verwendet wobei die Flüssigkeitszufuhr 5 ml/min betrug. Das Zerstäubungsgas war Sauerstoff (5 l/min). Die Stützflamme wurde mit vorgemischtem Methan/Sauerstoff (1,5 l/min / 3,2 l/min) betrieben. Der Hülluftstrom war 5 l/min Sauerstoff. Als Precursor für Si bzw. Yb wurde Tetraethoxysilan (TEOS) bzw. Ytterbiumnitrat-Pentahydrat (Yb(NO)_{3*}5H₂O) verwendet. Um sämtliches Kristallwasser aus dem Yb-Precursor zu entfernen, wurde Yb(NO)₃·5H₂O in 18,75 Vol.-% Essigsäureanhydrid und 81,25 Vol.-% 2-Ethylhexansäure unter Inertgas (N₂) bei 107°C umgesetzt. Dabei wurden sämtliche Stickoxide ausgetrieben und das Wasser durch seine Reaktion mit Essigsäureanhydrid zu Essigsäure entfernt. Diese so gebildete Lösung wurde mit 45,08 Vol.-% Xylol und TEOS vermischt, wobei eine Gesamtmetallkonzentration von 0,5 mol/l erreicht wurde. Mit dieser Methode konnten einfach verschiedene Yb/(Yb+Si)-Verhältnisse eingestellt werden. Damit waren Lösungen bis zu 50 Masse-% an Yb₂O₃, in Bezug zum nominalen Gesamtoxidgewicht, möglich. Für Lösungen mit einem nominalen Ytterbiumoxidgehalt von deutlich kleiner als 50 Masse-% wurde mit einer Mischung aus Essigsäureanhydrid und 2-Ethylhexansäure (ca. 3:13 nach Volumen) verdünnt. Dies ermöglichte die Änderung der Yb-Konzentration, ohne die Gesamtenthalpie der Lösung zu ändern. Das hergestellte Pulver zeichnete sich durch kugelförmige Gestalt der Primärpartikel aus (siehe Figur 1).

Desweiteren wurde eine homogene atomare Verteilung von Si- und Yb-Atomen im Mischoxid erreicht (siehe Figur 2). Dies konnte mit Hilfe der Infrarotspektroskopie bestätigt werden (siehe Figur 3). Alle hergestellten Pulver waren röntgenamorph was durch XRD-Messungen bestätigt wurde (siehe Figur 4). Der Brechungsindex der Mischoxidpulver konnte mit Hilfe des Ytterbiumgehalts sehr genau eingestellt werden. Tabelle 1 zeigt Beispiele für 10 bis 50 Masse-% Yb₂O₃ mit Brechungsindices von 1.449 bis 1.560. Es konnte gezeigt werden, daß diese Pulver Appen's Gesetz folgen, d.h. linearer Anstieg mit atomaren Anteil Yb (Tabelle 1).

**Tabelle 1: Experimentell bestimmte Brechungsindices von Yb/SiO₂ Mischoxiden**

| Yb₂O₃ (Masse-%) | Yb₂O₃ (Mol-%) | Brechungsindex |
|---|---|---|
| 10 | 3,3 | 1.499 |
| 20 | 7,1 | 1,513 |
| 30 | 11,5 | 1,523 |
| 40 | 16,9 | 1,539 |
| 50 | 23.3 | 1,560 |

Tabelle 2 zeigt beispielhaft wie mit Hilfe der Flammencharakteristik, hier Änderung der Menge an Zerstäubungsluft (Reduktion von 5 l/min auf 3 l/min) und Flüssigkeitszufuhr (Erhöhung von 5 ml/min auf 8 ml/min), die spezifische Oberfläche der Pulver deutlich verändert bzw. eingestellt werden kann. Der Brechungsindex des Pulvers wurde dabei nahezu nicht beeinflußt.

**Tabelle 2: Änderung der spezifischen Oberfläche der Yb/Si-Mischoxidpulver durch Variation der Sprühflammenparameter**

| Zerstäubungs-gas (l/(min) | Fördermenge Flüssigkeit (ml/min) | Spezifische Oberfläche (m²/g) | Brechungsindex |
|---|---|---|---|
| 5 | 5 | 245 | 1.530 |
| 8 | 3 | 135 | 1.523 |

### Beispiel 2: Evaluierung der Verdickungswirkung der nach Beispiel 1 hergestellten Mischoxide der Elemente Si und Yb

Zur Untersuchung der Verdickungswirkung wurden Modellkompositpasten hergestellt aus 16,5% Yb/Si-Mischoxid verschiedener spezifischer Oberfläche und 83,5 % radikalisch polymerisierbare Monomerzusammensetzung (41,82 Teile Bis-GMA, 37 Teile UDMA, 20 Teile TEGDMA, 0,73 Teile Photoinitiator, 0,55 Teile Additive). Zwischen zwei Glasplatten wurden 0,1g Paste mit einer Last von 120 g über einen Zeitraum von drei Minuten belastet und der sich dann ergebende Durchmesser der Paste bestimmt. Je dünner diese sogenannte Scheibenkonsistenz der Paste, also je geringer die Verdickungswirkung, umso größer ist der sich ergebene Durchmesser. Die Ergebnisse belegen, daß die Konsistenz der Pasten deutlich von der spezifischen Oberfläche des Nanopartikelfüllstoffs abhängt (Tabelle 3).

**Tabelle 3: Einfluß der spezifischen Oberfläche des Mischoxid-Füllstoffs auf die Scheibenkonsistenz der Kompositpasten**

| Spezifische Oberfläche (m²/g) | Scheibenkonsistenz (mm) |
|---|---|
| 399 | 11,7 |
| 365 | 18,5 |
| 291 | 24,4 |
| 233 | 33,3 |
| 205 | 36,0 |
| 125 | 38,1 |

Durch Agglomeratbildung läßt sich die Verdickungswirkung verringern. Die Agglomerierung erfolgt zum Beispiel auf die Art, daß 37,5% entionisiertes Wasser vorgelegt werden und 62,4% Mischoxid, sowie 0,16% Kaliumfluorozirkonat langsam eingerührt wird, bis eine homogene Suspension erreicht wird. Diese Suspension wird 30 Stunden bei 120°C getrocknet, in einer Kugelmühle gemahlen und gesiebt. Danach kann der so agglomerierte Füllstoff für die Herstellung eines Komposites eingesetzt werden.

### Beispiel 3: Herstellung eines Füllungskomposites auf der Basis eines nach Beispiel 1 hergestellten Mischoxides der Elemente Si und Yb

Es wurde ein Komposit (Komposit A) auf der Basis von 48 Masse-% an lichthärtender Monomerzusammensetzung (41,82 Teile Bis-GMA, 37 Teile UDMA, 20 Teile TEGDMA, 0,73 Teile Photoinitiator, 0,55 Teile) und 52 Masse-% eines nach Beispiel 1 durch Sprühflammensynthese hergestellten röntgenopaken Yb/Si-Mischoxides mit einem Gehalt an Yb₂O₃ von 30 Masse-% und einer spezifischen Oberfläche von 125 m²/g hergestellt. Als Vergleichsbeispiel wurde ein Komposit (Komposit B) auf der Basis von 50 Masse-% an dem gleichen lichthärtenden Monomer, 35 Masse-% von silanisiertem pyrogenem SiO₂ OX-50 und 15 Masse-% an Ytterbiumfluorid hergestellt und nach Aushärtung der Komposite die Transparenz und Röntgenopazität bestimmt:

**Tabelle 4: Transparenz und Röntgenopazität der Komposite aus Beispiel 3**

| Material | Transparenz (%) | Röntgenopazität (% Al) |
|---|---|---|
| Kom posit A | 13,2 | 180 |
| Komposit B | 9,5 | 150 |

Die Ergebnisse zeigen, daß mit dem röntgenopaken Yb/Si-Mischoxid eine akzeptable Transparenz und Röntgenopazität erreicht wurde. Um mit konventionellen Nanofüllern wie OX-50 ein Komposit mit vergleichbarer Röntgenopazität zu erreichen, ist der Zusatz von über 15% Ytterbiumfluorid notwendig. Damit liegen jedoch zwei Füller mit unterschiedlichem Brechungsindex vor, was sich deutlich negativ auf die Transparenz auswirkt.

### Beispiel 4: Herstellung eines Füllungskomposits auf der Basis eines vorpolymerisierten Füllstoffs ausgehend von einem nach Beispiel 1 hergestellten Mischoxid

Zunächst wurde ein vorpolymerisierter Füllstoff (Präpolymer) hergestellt. Dazu wurde ausgehend von 70 g nach Beispiel 1 durch Sprühflammensynthese hergestelltem röntgenopaken Yb/Si-Mischoxid (30 Masse-% Yb₂O₃ und spezifische Oberfläche von 125 m²/g) und 30 g heißhärtender Monomerzusammensetzung (80% Decandioldimethacrylat, 12% UDMA, 8% Dibenzoylperoxid) eine homogene Mischung hergestellt, die dann 1 Stunde bei 120°C polymerisiert und auf eine Korngröße von 10-20 µm gemahlen wurde. Mit diesem Präpolymer (44 Masse-%), 24 Masse-% an lichthärtender Monomerzusammensetzung (41,82 Teile Bis-GMA, 37 Teile UDMA, 20 Teile TEGDMA, 0,73 Teile Photoinitiator, 0,55 Teile Additive) und weiteren 33 Masse-% an nach Beispiel 1 durch Sprühflammensynthese hergestelltem röntgenopaken Yb/Si-Mischoxid (30 Masse-% Yb₂O₃ und spezifische Oberfläche von 125 m²/g) wurde schließlich eine Kompositpaste hergestellt. Daraus wurden Prüfkörper präpariert, die 2x3 Minuten im Lichtofen Spectramat (Ivoclar Vivadent) ausgehärtet und 24 Stunden bei 37°C in Wasser gelagert wurden. Damit wurden folgende Eigenschaften bestimmt:

| | |
|---|---|
| Biegefestigkeit | 110 MPa |
| Biege-E-Modul | 6500 MPa |
| Transparenz | 14 % |
| Röntgenopazität | 300 % Al |

### Beispiel 5: Herstellung eines Füllungskomposits auf der Basis eines Yb/Si-Mischoxides und eines konventionellen Glasfüllers

Es wurde ein Komposit auf der Basis von 20 Masse-% an lichthärtender Monomer zusammensetzung (41,82 Teile Bis-GMA, 37 Teile UDMA, 20 Teile TEGDMA, 0,73 Teile Photoinitiator, 0,55 Teile Additive), 40 Masse-% eines nach Beispiel 1 durch Sprühflammensynthese hergestellten röntgenopaken Yb/Si-Mischoxides (30 Masse-% Yb₂O₃ und spezifische Oberfläche von 125 m²/g), und 40 Masse-% eines silanisierten Ba-Al-Silikatglases (GM 27884 der Firma Schott) mit einer mittleren Partikelgröße von 1,0 µm hergestellt. Daraus wurden Prüfkörper präpariert, die 2x3 Minuten im Lichtofen Spectramat (Ivoclar Vivadent) ausgehärtet und 24 h bei 37°C in Wasser gelagert wurden. Damit wurden folgende Eigenschaften bestimmt:

| | |
|---|---|
| Biegefestigkeit | 130 MPa |
| Biege-E-Modul | 9000 MPa |
| Transparenz | 14 % |
| Röntgenopazität | 400 % Al |

Die vorliegenden Ergebnisse belegen, daß der röntgenopake Yb₂O₃/SiO₂-Nanofüllstoff mit einem Brechungsindex von 1.53 zum Beispiel mit Ba-Al-Silikatgläsern kombiniert werden kann, ohne daß Einbußen in der Transparenz sichtbar werden. Demgegenüber führt die Kombination von SiO₂-Nanofüllem gemäß dem Stand der Technik mit röntgenopaken Gläsern meist zu Defiziten in der Transparenz, da die Brechungsindices der beiden Füller weit auseinander liegen und nicht deckungsgleich sind.

## Patentansprüche

1. Dentalkomposite enthaltend
mindestens ein durch Sprühflammensynthese erhältliches nanopartikuläres Mischoxid (a) von SiO₂ mit röntgenopaken Metalloxiden eines oder mehrerer Elemente ausgewählt aus der Gruppe bestehend aus Y, La, Ta, Ce, Pr, Nd, Sm, Eu, Gd, Tb. Dy, Ho, Er, Tm, Yb, Lu.

2. Dentalkomposite nach Anspruch 1, wobei die Mischoxide mindestens eines, insbesondere alle, der folgenden Merkmale erfüllen:
a) amorphe Struktur,
b) homogene Elementverteilung,
c) sehr geringer organischer Anteil,
d) variierbare Röntgenopazität,
e) variierbarer Brechungsindex,
f) kugelförmige Partikelgestalt.

3. Dentalkomposite nach Anspruch 1 oder 2, wobei die Mischoxide (a) Mischoxide von SiO₂ mit einem röntgenopaken Metalloxid von Yb sind.

4. Dentalkomposite nach einem der Ansprüche 1 bis 3 enthaltend ein Mischoxid (a).

5. Dentalkomposite nach einem der Ansprüche 1 bis 4, **dadurch gekennzeichnet, daß**
(a) 5 bis 90 Gew.-%, bezogen auf die Zusammensetzung, Mischoxide enthalten sind.

6. Dentalkomposite nach Anspruch 5, **dadurch gekennzeichnet, daß** zusätzlich
(b) 0 bis 80, bezogen auf die Zusammensetzung, mindestens ein Matrixmonomer,
(c) 0.1 bis 5, bezogen auf die Zusammensetzung, Polymerisationsinitiator, und/oder
(d) 0 bis 90, bezogen auf die Zusammensetzung an weiteren Füllstoffen,
(e) 0.0001 bis 0,5, bezogen auf die Zusammensetzung. Farbmittel,
(f) 0,001 bis 2,0, bezogen auf die Zusammensetzung, weitere Additive
enthalten sind,
wobei sich die Anteile der Komponenten (a) bis (f) zu 100 Gew.-% addieren.

7. Dentalkomposite nach einem der Ansprüche 1 bis 4,**dadurch gekennzeichnet, daß** sie aus
(a) 5 bis 90, bezogen auf die Zusammensetzung, Mischoxiden (a),
(b) 0 bis 80, bezogen auf die Zusammensetzung, mindestens ein Matrixmonomer,
(c) 0,1 bis 5, bezogen auf die Zusammensetzung, Polymerisationsinitiator, und/oder
(d) 0 bis 90, bezogen auf die Zusammensetzung an weiteren Füllstoffen,
wobei sich die Anteile der Komponenten (a) bis (d) zu 100 Gew.-% addieren, bestehen.

8. Dentalkomposite nach einem der Ansprüche 1 bis 7, **dadurch gekennzeichnet, daß**
sie eine Röntgenopazität von 50 bis 800% Al aufweisen, und daß
die Mischoxide (a) eine mittlere Primärpartikelgröße von 3 bis 100nm, bestimmt durch Messung der BET-Oberfläche,
einen Brechungsindex von 1,46 bis 1,70 aufweisen, und/oder
einen organischen Anteil von weniger als 0,5 Masse-%, oxidierbaren Kohlenstoff, aufweisen.

9. Verfahren zur Herstellung von Dentalkompositen nach einem der Ansprüche 1 bis 8, **dadurch gekennzeichnet, daß**
(i) nanopartikuläre Mischoxide (a) von SiO₂ mit röntgenopaken Metalloxide eines oder mehrerer Elemente ausgewählt aus der Gruppe Y. La, Ta, Ce, Pr, Nd, Sm, Eu, Gd, Tb, Dy. Ho, Er, Tm, Yb, Lu, durch Sprühflammensynthese hergestellt werden,
(ii) die Mischoxide (a) in geeigneten polymerisierbaren Matrixharzen dispergiert,
(iii) mit einem Polymerisationsinitiator (c) versetzt,
(iv) gegebenenfalls mit weiteren Bestandteilen (b), (d), (e) und/oder (f) vermischt und
(v) anschließend durch thermische oder lichtinduzierte Polymerisation ausgehärtet wird.

10. Verwendung der Dentalkomposite nach einem der Ansprüche 1 bis 8 zur Herstellung von Dentalfüllungen, zur Herstellung von Oberflächenversiegelung von Zähnen, sowie zur Herstellung eines Materials zur Restauration von Zähnen.

11. Verwendung der Dentalkomposite nach einem der Ansprüche 1. bis 8 zur Herstellung von Zahnprothesen; Dentalbrücken und/oder ähnlichen Zahnersatzstücken.

12. Verwendung nach Anspruch 10 oder 11, **dadurch gekennzeichnet, daß** die Dentalkomposite in eine Form, eingebracht und gehärtet werden

13. Verwendung der Dentalkomposite nach einem der Ansprüche 1 bis 8 zur Herstellung von Füllung oder Beschichtung von Substraten.

14. Verwendung der Dentalkomposite nach einem der Ansprüche 1 bis 8 zur Herstellung von Adhäsiv, Zement und/oder Verblendmateriaüen Zement für Dentalanwendungen.

15. Verwendung eines durch Sprühflammensynthese hergestellten nanopartikulären Mischoxids von SiO₂ mit röntgenopaken Metalloxiden eines oder mehrerer Elemente ausgewählt aus der Gruppe bestehend aus Y, La, Ta, Ce, Pr, Nd, Sm, Eu, Gd, Tb, Dy, Ho, Er, Tm, Yb, Lu als röntgenopaker Füllstoff.

## Claims

1. Dental composites comprising at least one nanoparticulate mixed oxide (a) of SiO₂ with X-ray-opaque metal oxides of one or more elements selected from the group consisting of Y, La, Ta, Ce, Pr, Nd, Sm, Eu, Gd, Tb, Dy, Ho, Er, Tm, Yb, Lu which can be obtained by flame spraying synthesis.

2. Dental composites according to Claim 1, wherein the mixed oxides have at least one, in particular all, of the following features:
a) an amorphous structure,
b) a homogeneous element distribution,
c) a very low organic content,
d) an X-ray opacity which can be varied,
e) an index of refraction which can be varied,
f) a spherical particle shape.

3. Dental composites according to Claim 1 or 2, wherein the mixed oxides (a) are mixed oxides of SiO₂ with an X-ray-opaque metal oxide of Yb.

4. Dental composites according to any of Claims 1 to 3 comprising a mixed oxide (a).

5. Dental composites according to any of Claims 1 to 4, **characterized in that**
(a) from 5 to 90% by weight, based on the composition, of mixed oxides are present.

6. Dental composites according to Claim 5, **characterized in that**
(b) from 0 to 80% by weight, based on the composition, of at least one matrix monomer,
(c) from 0.1 to 5% by weight, based on the composition, of polymerization initiator, and/or
(d) from 0 to 90% by weight, based on the composition, of further fillers,
(e) from 0.0001 to 0.5% by weight, based on the composition, of colorants,
(f) from 0.001 to 2.0% by weight, based on the composition, of further additives,
are additionally present,
where the proportions of the components (a) to (f) add up to 100% by weight.

7. Dental composites according to any of Claims 1 to 4, **characterized in that** they consist of
(a) from 5 to 90% by weight, based on the composition, of mixed oxides (a),
(b) from 0 to 80% by weight, based on the composition, of at least one matrix monomer,
(c) from 0.1 to 5% by weight, based on the composition, of polymerization initiator, and/or
(d) from 0 to 90% by weight, based on the composition, of further fillers,
where the proportions of the components (a) to (d) add up to 100% by weight.

8. Dental composites according to any of Claims 1 to 7, **characterized in that** they have
an X-ray opacity of from 50 to 800% Al, and **in that**
the mixed oxides (a) have a mean primary particle size of from 3 to 100 nm, determined by measurement of the BET surface area,
have an index of refraction of from 1.46 to 1.70, and/or
have an organic content of less than 0.5% by mass of oxidizable carbon.

9. Process for producing dental composites according to any of Claims 1 to 8,
**characterized in that**
(i) nanoparticulate mixed oxides (a) of SiO₂ with X-ray-opaque metal oxides of one or more elements selected from the group consisting ofY, La, Ta, Ce, Pr, Nd, Sm, Eu, Gd, Tb, Dy, Ho, Er, Tm, Yb, Lu, are prepared by flame spraying synthesis,
(ii) the mixed oxides (a) are dispersed in suitable polymerizable matrix resins,
(iii)a polymerization initiator (c) is added,
(iv) if desired, the dispersion is mixed with further constituents (b), (d), (e) and/or (f) and
(v) the dispersion is subsequently cured by thermal or light-induced polymerization.

10. Use of the dental composites according to any of Claims 1 to 8 for producing dental fillings, for producing surface sealing of teeth and for producing a material for the restoration of teeth.

11. Use of the dental composites according to any of Claims 1 to 8 for producing dental prostheses, dental bridges and/or similar tooth replacement pieces.

12. Use according to Claim 10 or 11, **characterized in that** the dental composites are introduced into a mold and cured.

13. Use of the dental composites according to any of Claims 1 to 8 for producing filling or coating of substrates.

14. Use of the dental composites according to any of Claims 1 to 8 for producing adhesive, cement and/or facing material cement for dental applications.

15. Use of a nanoparticulate mixed oxide of SiO₂ with X-ray-opaque metal oxides of one or more elements selected from the group consisting of Y, La, Ta, Ce, Pr, Nd, Sm, Eu, Gd, Tb, Dy, Ho, Er, Tm, Yb, Lu which has been prepared by flame spraying synthesis as an X-ray-opaque filler.

## Revendications

1. Composites dentaires contenant au moins un oxyde mixte nanoparticulaire pouvant être obtenu par synthèse par projection à la flamme (a) de SiO₂ avec des oxydes de métal opaques aux rayons X d'un ou de plusieurs éléments choisis dans le groupe constitué par Y, La, Ta, Ce, Pr, Nd, Sm, Eu, Gd, Tb, Dy, Ho, Er, Tm, Yb, Lu.

2. Composites dentaires selon la revendication 1, les oxydes mixtes remplissant au moins une des caractéristiques suivantes, en particulier toutes :
a) structure amorphe,
b) répartition homogène des éléments,
c) proportion organique très basse,
d) opacité aux rayons X pouvant être variée,
e) indice de réfraction pouvant être varié,
f) aspect sphérique des particules.

3. Composites dentaires selon la revendication 1 ou 2, les oxydes mixtes (a) étant des oxydes mixtes de SiO₂ avec un oxyde métallique opaque aux rayons X d'Yb.

4. Composites dentaires selon l'une quelconque des revendications 1 à 3 contenant un oxyde mixte (a).

5. Composites dentaires selon l'une quelconque des revendications 1 à 4, **caractérisés en ce qu'**ils contiennent
(a) 5 à 90% en poids, par rapport à la composition, d'oxydes mixtes.

6. Composites dentaires selon la revendication 5, **caractérisés en ce qu'**ils contiennent en outre
(b) 0 à 80% en poids, par rapport à la composition, d'au moins un monomère formant une matrice,
(c) 0,1 à 5% en poids, par rapport à la composition, d'initiateur de polymérisation, et/ou
(d) 0 à 90% en poids, par rapport à la composition, d'autres charges,
(e) 0,0001 à 0,5% en poids, par rapport à la composition, de colorants,
(f) 0,001 à 2,0% en poids, par rapport à la composition, d'autres additifs,
les proportions des composants (a) à (f) s'additionnant à 100% en poids.

7. Composites dentaires selon l'une quelconque des revendications 1 à 4, **caractérisés en ce qu'**ils sont constitués par
(a) 5 à 90% en poids, par rapport à la composition, d'oxydes mixtes (a),
(b) 0 à 80% en poids, par rapport à la composition, d'au moins un monomère formant une matrice,
(c) 0,1 à 5% en poids, par rapport à la composition, d'initiateur de polymérisation, et/ou
(d) 0 à 90% en poids, par rapport à la composition, d'autres charges,
les proportions des composants (a) à (d) s'additionnant à 100% en poids.

8. Composites dentaires selon l'une quelconque des revendications 1 à 7, **caractérisés en ce qu'**ils présentent une opacité aux rayons X de 50 à 800% d'Al, et
**en ce que** les oxydes mixtes (a) présentent une grosseur moyenne des particules primaires de 3 à 100 nm, déterminée par mesure de la surface BET, un indice de réfraction de 1,46 à 1,70, et/ou une proportion organique inférieure à 0,5% en masse, de carbone oxydable.

9. Procédé pour la préparation de composites dentaires selon l'une quelconque des revendications 1 à 8, **caractérisé en ce qu'**on
(i) prépare des oxydes mixtes nanoparticulaires (a) de SiO₂ avec des oxydes de métal opaques aux rayons X d'un ou de plusieurs éléments choisis dans le groupe constitué par Y, La, Ta, Ce, Pr, Nd, Sm, Eu, Gd, Tb, Dy, Ho, Er, Tm, Yb, Lu par synthèse par projection à la flamme,
(ii) disperse les oxydes mixtes (a) dans des résines formant une matrice polymérisables appropriées,
(iii) ajoute un initiateur de polymérisation (c),
(iv) mélange le cas échéant avec d'autres constituants (b), (d), (e) et/ou (f) et
(v) durcit ensuite par polymérisation thermique ou induite par la lumière.

10. Utilisation des composites dentaires selon l'une quelconque des revendications 1 à 8 pour la préparation de remplissages dentaires, pour la préparation de scellements de surface de dents ainsi que pour la préparation d'un matériau destiné à la restauration de dents.

11. Utilisation des composites dentaires selon l'une quelconque des revendications 1 à 8 pour la préparation de prothèses dentaires ; de bridges dentaires et/ou de parties des prothèses dentaires analogues.

12. Utilisation selon la revendication 10 ou 11, **caractérisée en ce que** les composites dentaires sont introduits dans un moule et durcis.

13. Utilisation des composites dentaires selon l'une quelconque des revendications 1 à 8 pour la préparation de substances de remplissage ou de revêtement de substrats.

14. Utilisation des composites dentaires selon l'une quelconque des revendications 1 à 8 pour la préparation d'un adhésif, d'un ciment et/ou de matériaux de mélange au ciment pour utilisations dentaires.

15. Utilisation d'un oxyde mixte nanoparticulaire préparé par synthèse par projection à la flamme de SiO₂ avec des oxydes de métal opaques aux rayons X d'un ou de plusieurs éléments choisis dans le groupe constitué par Y, La, Ta, Ce, Pr, Nd, Sm, Eu, Gd, Tb, Dy, Ho, Er, Tm, Yb, Lu comme substance de remplissage opaque aux rayons X.
